# EUROPÄISCHE PATENTSCHRIFT

(11) **EP 2 569 283 B1**
(45) Veröffentlichungstag und Bekanntmachung des Hinweises auf die Patenterteilung: **27.08.2014**
(21) Anmeldenummer: 11717663.6
(22) Anmeldetag: 06.05.2011
(51) Int. Cl.: C07D 209/34

(54) **VERFAHREN ZUR HERSTELLUNG VON SUBSTITUIERTEN 1,3-DIHYDRO-2H-INDOL-2-ONEN**
METHOD FOR PRODUCING SUBSTITUTED 1,3-DIHYDRO-2H-INDOL-2-ONES
PROCÉDÉ DE FABRICATION DE 1,3-DIHYDRO-2H-INDOL-2-ONS SUBSTITUÉS

(30) Priorität: 10.05.2010 US 332949 P; 10.05.2010 EP 10162381
(43) Veröffentlichungstag der Anmeldung: 20.03.2013
(73) Patentinhaber: Bayer Intellectual Property GmbH, 40789 Monheim (DE)
(72) Erfinder: SIEGEL, Konrad, 40597 Düsseldorf (DE); KARIG, Gunter, 65719 Hofheim am Taunus (DE)
(86) Internationale Anmeldenummer: PCT/EP2011/057276
(87) Internationale Veröffentlichungsnummer: WO 2011/141364

(56) Entgegenhaltungen:
- CONNOLLY T J ET AL: "Non-reductive desulfenylation of 3-thioalkyl-2-oxindoles", SYNLETT, GEORG THIEME VERLAG, DE, 1. Juli 1996 (1996-07-01), Seiten 663-664, XP002545539, ISSN: 0936-5214

## Beschreibung

1,3-Dihydro-2*H*-indol-2-one der Formel (I) 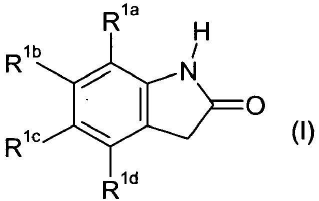 sind Zwischenprodukte für Wirkstoffe, die bei der Bereitstellung von Arznei- oder Pflanzenschutzmittel eingesetzt werden.

Die Herstellung von Verbindungen der Formel (I) kann z. B. durch Reduktion von 3 (-Alkylsulfanyl)-1,3-dihydro-2H-indol-2-onen erfolgen. Letztere lassen sich im Labormaßstab gut herstellen. Jedoch sind für deren Reduktion bislang nur Verfahren beschrieben, die sich schlecht im technischen Maßstab handhaben lassen. Diese sind teuer und können außerdem auch sicherheitstechnisch anspruchsvoll sein.

Aus dem Stand der Technik sind bereits durch Metalle oder durch Metallsalze katalysierte Verfahren zur Umsetzung von thioalkyl-substituierten Indol-2-on Verbindungen der Formel (II) zu den in 3-Position unsubstituierten Indolverbindungen der Formel (I) bekannt. Die nachfolgelnde Zusammenstellung dieser bekannten, aber lediglich für den Labormaßstab tauglichen, Reduktionsmethoden dokumentiert, dass der Entwicklung geeigneter Methoden in diesem Bereich ein sich bereits über einen längeren Zeitraum erstreckendes Bemühen zugrunde liegt.

Die in den nachfolgend aufgelisteten, aus dem Stand der Technik bekannten, Verfahren eingesetzten Reagenzien sind jeweils in Klammern angegeben:
- P.G. Grassmann, T.J van Bergen, Journal of the American Chemical Society 1973, S. 2718-2719 (Raney-Nickel),
- US-Patentschrift 4,160,032, basierend auf einer Anmeldung, die im Jahr 1974 eingereicht worden war, (Palladium und Raney-Nickel),
- D. A. Walsh, D. A. Shamblee, W. J. Welstead, L. F. Sancilio, J. Med. Chem 1982, 25, 446 - 451 (Raney-Nickel),
- Y. Tamura, J. Uenishi, H. D. Choi, J. Haruta, H. Ishibashi, Chem. Pharm. Bull. 1984, 32, 1995 - 1997 (Zink-Pulver in Essigsäure),
- D. A. Walsh, D. A. Shamblee, US-Patentschrift 4,503,073 (Zinn in Salzsäure anstatt Raney-Nickel),
- Y. Horiguchi, A. Sonobe, T. Saitoh, J. Toda, T. Sano, Chem. Pharm. Bull. 2001, 49, 1132 - 1137 (NiCl₂ und NaBH₄) und
- M. Miller, W. Tsang, A. Merritt, D. J. Procter, Chem. Commun. 2007, 498 - 500 (Sml₂).

Für die gleiche Umsetzung, d.h. die Umsetzung von thioalkyl-substituierten Indol-2-on Verbindungen der Formel (II) zu den in 3-Position unsubstituierten Indolverbindungen der Formel (I), wird in T. J. Connolly, T. Durst, Synlett 1996, 663-664 offenbart, dass als Reagenz auch PPh₃ in Kombination mit p-TsOH eingesetzt werden kann.

Somit benutzen die bekannten Verfahren entweder ein schwermetallhaltiges Reagenz oder Triphenylphosphin. Im ersten Fall entstehen schwermetallhaltige Abfälle. Aus ökologischen und ökonomischen Gründen ist dies nicht wünschenswert. Im zweiten Fall muß das aus dem Triphenylphosphin entstehende Reaktionsprodukt vom Reaktionsgemisch abgetrennt werden. Diese Abtrennung ist unter zusätzlichem Aufwand meist nur durch Chromatographie möglich.

Außerdem hat das jeweilige Reagenz eine hohe Molmasse und muss jedoch stöchiometrisch eingesetzt werden. Dies ist aus ökonomischen Gründen nachteilig.

Die aus dem Stand der Technik bekannten Verfahren sind allerdings lediglich im Labormaßstab dienlich und weisen auch aufgrund der chemischen Eigenschaften der als Reagenz eingesetzten Metallverbindungen Nachteile auf, wobei sich die Nachteile bei Anwendung der Verfahren im betrieblichen Maßstab noch stärker auswirken.

Vor diesem Hintergrund besteht die Aufgabe der Erfindung in der Entwicklung eines Verfahrens, das die Herstellung der Verbindung der Formel (I) im betrieblichen, Maßstab erlaubt und nicht die Nachteile der aus dem Stand der Technik bekannten Verfahren aufweist.

Gelöst wird die Aufgabe durch ein einstufiges Verfahren zur Herstellung einer Verbindung der Formel (I) 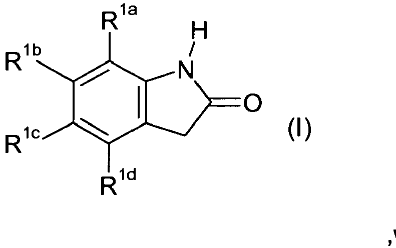 worin
R^{1a} bis R^{1d} unabhängig voneinander ausgewählt sind aus der Gruppe bestehend aus Wasserstoff, Fluor, Chlor, Brom und Trifluormethyl,
(C₁-C₆)-Alkyl, wobei der Alkylrest unsubstituiert ist oder durch einen oder mehrere Substituenten, ausgewählt aus der aus (C₁-C₄)-Alkoxy oder (C₃-C₇)-Cycloalkyl bestehenden Gruppe, substituiert ist,
(C₃-C₇)-Cycloalkyl, wobei der Cycloalkylrest unsubstituiert ist oder durch einen oder mehrere Substituenten, ausgewählt aus der aus (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest verzweigt oder unverzweigt unsubstituiert ist oder durch einen oder mehrere Substituenten, ausgewählt aus der aus (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus (C₁-C₄)-Alkyl oder (C₁-C₄)-Alkoxy bestehenden Gruppe, substituiert ist,
(C₁-C₆)-Alkylthio, wobei der Alkylthiorest verzweigt oder unverzweigt unsubstituiert ist oder durch einen oder mehrere Substituenten, ausgewählt aus der aus (C₁-C₄)-Alkyl oder (C₁-C₄)-Alkoxy bestehenden Gruppe, substituiert ist,
(C₃-C₇)-Cycloalkylthio, wobei der Cycloalkylthiorest unsubstituiert ist oder durch einen oder mehrere Substituenten, ausgewählt aus der aus aus (C₁-C₄)-Alkyl oder (C₁-C₄)-Alkoxy bestehenden Gruppe, substituiert ist, und
Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus (C₁-C₄)-Alkyl, (C₁-C₄)-Alkoxy oder (C₃-C₇)-Cycloalkyl oder (C₁-C₄)-Alkylthio bestehenden Gruppe, substituiert ist,
ausgehend von einer Verbindung der Formel (II) 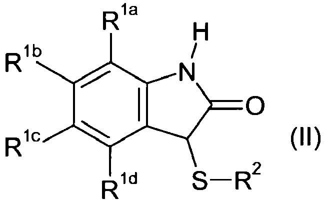 worin
R^{1a} bis R^{1d} wie in Formel (I) definiert ist, und
R² ein unsubstituiertes oder substituiertes (C₁-C₁₄)-Alkyl, (C₃-C₇)-Cycloalkyl, Benzyl oder ein CH₂-C(O)O-(C₁-C₆)-Alkyl ist,
dadurch gekennzeichnet, dass
a) eine Verbindung der Formel (II) in einem polaren Lösungsmittel gelöst oder suspendiert wird,
b) der Lösung oder der Suspension ein schwefelhaltiges Salz zugesetzt wird, und
c) das Reaktionsgemisch bei einer Temperatur, die maximal der Siedetemparatur des polaren Lösungsmittels entspricht, unter Rückfluss erwärmt wird.

Das erfindungsgemäße Verfahren erlaubt eine einfache, sichere und kostengünstige Herstellung von Verbindungen der Formel (I) aus Verbindungen der Formel (II).

Das Verfahren lässt sich auch im betrieblichen Maßstab sicher durchführen. Vorteilhafterweise lässt sich das Verfahren außerdem flexibel an die apparativen Voraussetzungen anpassen.

Besonders überraschend und sehr vorteilhaft ist, dass bei dem erfindungsgemäßen Verfahren keine geruchsintensiven Abgase entstehen, wie sie für eine solche Reaktion eigentlich zu erwarten sind. Dieser Vorteil beruht vermutlich darauf, dass die schwefelhaltige Abgangsgruppe in einer nicht flüchtigen Form gebunden wird.

Bevorzugt als Phenylsubstituenten R^{1a}, R^{1b}, R^{1c} und R^{1d} sind Reste die unabhängig voneinander ausgewählt sind aus der Gruppe bestehend aus Wasserstoff, (C₁-C₆)-Alkyl, (C₁-C₆)-Alkoxy, Fluor, Chlor, Brom und Trifluormethyl.

Besonders bevorzugt als Phenylsubstituenten R^{1a}, R^{1b}, R^{1c} und R^{1d} sind Reste die unabhängig voneinander ausgewählt sind aus der Gruppe bestehend aus Wasserstoff, Methoxy, Fluor und Chlor.

Bevorzugt für den Rest R² ist unsubstituiertes oder substituiertes (C₁-C₆)-Alkyl, (C₃-C₇)-Ccyloalkyl, Benzyl oder CH₂-C(O)O-(C₁-C₆)-Alkyl, wobei die Substituenten unabhängig voneinander ausgewählt sind aus der Gruppe bestehend aus Wasserstoff, (C₁-C₆)-Alkyl oder (C₃-C₇)-Cycloalkyl.

Bevorzugt als polares Lösungsmittel ist Wasser, ein (C₁-C₄) Alkohol oder ein Gemisch aus Wasser und einem (C₁-C₄) Alkohol. Dabei ist auch ein Gemisch aus Wasser und einem unter den Reaktionsbedingungen stabilen anderen polaren Lösungsmittel als einem (C₁-C₄) Alkohol einsetzbar. Das polare Lösungsmittel löst das als Reduktionsmittel wirkende schwefelhaltige Salz ganz oder zumindest teilweise auf.

In einer weiteren bevorzugten Ausführungsform ist das schwefelhaltige Salz ausgewählt aus der Gruppe bestehend aus Alkali- oder Erdalkalisulfit, Alkali- oder Erdalkalibisulfit, Alkali- oder Erdalkalithionit, Alkali- oder Erdalkalidithionit oder Alkali- oder Erdalkalithiosulfat.

Ganz besonderes bevorzugt als schwefelhaltige Salze sind die jeweiligen Natriumsalze, d.h. das schwefelhaltige Salz ist ausgewählt aus der Gruppe bestehend aus Natriumbisulfit, Natriumsulfit, Natriumthionit, Natriumdithionit und Natriumthiosulfat.

Am meisten bevorzugt als schwefelhaltige Salze sind Natriumbisulfit oder Natriumsulfit, wobei auch ein Gemisch aus beiden eingesetzt werden kann.

Falls als schwefelhaltiges Salz Natriumbisulfit eingesetzt wird, ist Wasser das bevorzugte polare Lösungsmittel, weil diese Kombination von Reagenz und Lösungsmittel zu sehr hohen Ausbeuten führt.

Bevorzugt ist die Zugabe von 2 bis 3 Äquivalenten des schwefelhaltigen Salzes zum Reaktionsgemisch.

Besonders bevorzugt ist die Zugabe von 2 bis 2,5 Äquivalenten des schwefelhaltigen Salzes zum Reaktionsgemisch.

In einer weiteren bevorzugten Ausführungsform ist vorgesehen, dass das Reaktionsgemisch auf eine Temperatur erwärmt wird, die in einem Bereich liegt, dessen untere Grenze 40° C ist und dessen obere Grenze der Rückflußtemperatur des Lösungsmittels entspricht.

Besonders bevorzugt ist jedoch die Ausführung des Verfahrens bei einer Reaktionstemperatur, welche etwa der Siedetemperatur des jeweils eingesetzten Lösungsmittels entspricht. Dabei kann die Durchführung der Reaktion auch unter erhöhtem Druck erfolgen, um eine Temperatur zu erreichen, die bis zu 15° C über dem Siedepunkt des jeweiligen Lösungsmittels bei Normaldruck liegt. Eine bis zu 15° C erhöhte Reaktionstemperatur ermöglicht vorteilhafterweise eine verkürzte Reaktionsdauer.

In einer bevorzugten Ausführungsform erfolgt die Erwärmung des Reaktionsgemisches unter Rühren für die Zeitdauer von 1 bis 48 Stunden. Besonders bevorzugt ist die Erwärmung des Reaktionsgemisches unter Rühren für die Zeitdauer von 1 bis 24 Stunden, wobei eine Reaktionszeit von 2 bis 12 Stunden am meisten bevorzugt ist.

Es liegt im Rahmen der Erfindung, dass dem Reaktionsgemisch vor dem Erreichen der Siedetemperatur des jeweils eingesetzten Lösungsmittels ein Entschäumer oder eine Mischung verschiedener Entschäumer zugesetzt wird. Geeignete Entschäumer sind beispielsweise Fluowet PL 80 oder Korasilon LP-Si E 1051.

Bevorzugt erfolgt die Zugabe des Entschäumers vor dem Beginn der Aufheizung des Reaktionsgemisches. Es liegt ebenfalls im Rahmen der Erfindung, dass dem Reaktionsgemisch vor dem Isolieren des Reaktionsproduktes ein Fällungsmittel zugesetzt wird, wobei das Fällungsmittel ausgewählt ist aus der Gruppe der polaren Lösungsmittel bestehend aus Wasser, einem (C₁-C₄) Alkohol oder einem Gemisch aus Wasser und einem (C₁-C₄) Alkohol. Die Zugabe des Fällungsmittels hat insbesondere den Vorteil, dass die Ausbeute bei der Isolierung durch Filtration erhöht wird.

Alternativ zur Isolierung durch Filtration kann das Reaktionsprodukt gemäß Formel (I) auch ohne Isolierung, z.B. als Suspension oder Lösung, weiter in der Synthese von Feinchemikalien und Wirkstoffen in der Pharmazie und/oder der Landwirtschaft eingesetzt werden.

Dementsprechend betrifft ein weiterer Aspekt die Verwendung einer Verbindung der Formel (I) zur Herstellung von Feinchemikalien und von Wirkstoffen mit pharmazeutischer oder herbizider Wirkung.

Weiterhin die Verwendung einer Verbindung der Formel (I) zur Herstellung von Wirkstoffen mit insektizider oder fungizider Wirkung.

Die nachfolgenden Beispiele erläutern die Erfindung näher.

### BEISPIELE

### Beispiel 1:

Herstellung von 7-Fluor-1,3-dihydro-2*H*-indol-2-on 7-Fluor-3-(methylsulfanyl)-1,3-dihydro-2*H*-indol-2-on (270,5 g) wurde in Wasser (2000 g) gelöst und mit Natriumbisulfit (300 g) versetzt. Die Mischung wurde auf Rückfluß erwärmt und dabei kräftig gerührt. Nach 2,25 h wurde die Mischung auf 25°C abgekühlt und über eine Nutsche durch Filtration isoliert. Es wurde zweimal mit Wasser (je 500 g) gewaschen und anschließend im Vakuum (<50 mbar, 50°C) getrocknet. Man erhielt 7-Fluor-1,3-dihydro-2*H*-indol-2-on als weißen Feststoff (206,3 g, 95% Ausbeute).
LC-MS: M+H = 152 (100%).
¹H-NMR (400 MHz, CDCl₃): δ (ppm) = 3.59 (s, 2H), 6.94-7.04 (m, 3H), 8.22 (s, breit, H).

### Beispiel 2:

### Herstellung von 5-Fluor-1,3-dihydro-2H-indol-2-on

5-Fluor-3-(methylsulfanyl)-1,3 -dihydro-2*H*-indol-2-on (20 g) wurde analog zu Beispiel 1 umgesetzt. Man erhielt 5-Fluor-1,3-dibydro-2*H*-indol-2-on als Feststoff (13.5 g, 93% Ausbeute).
LC-MS: M+H = 152 (100%).
¹H-NMR (400 MHz, CDCl₃): δ (ppm) = 3.55 (s, 2H), 6.78 (dd, 1 H), 6.89-7.00 (m, 2H), 8.32 (s, breit, H).

### Beispiel 3:

Herstellung von 7-Chlor-1,3-dihydro-2*H*-indol-2-on 7-Chlor-3-(methylsulfanyl)-1,3-dihydro-2*H*-indol-2-on (20 g) wurde analog zu Beispiel 1 umgesetzt. Man erhielt 7-Chlor-1,3-dihydro-2*H*-indol-2-on als Feststoff (15.6g, 99% Ausbeute).
LC-MS: M+H = 168 (100%), 170 (60%).
¹H-NMR (400 MHz, CDCl₃): δ (ppm) = 3.62 (s, 2H), 6.96 (t, 1H), 7.13 (d, 1 H), 7.22 (d, IH), 8.17 (s, breit, H).

### Beispiel 4:

Herstellung von 5,7-Difluor-1,3-dihydro-2*H*-indol-2-on 5,7-Difluor-3-(methylsulfanyl)-1,3-dihydro-2H-indol-2-on (4.25 g) wurde analog zu Beispiel 1 umgesetzt. Man erhielt die Titelverbindung als Feststoff (3.04 g, 91 % Ausbeute).
LC-MS: M+H = 170 (100%).
¹H-NMR (400 MHz, CDCl₃): δ (ppm) = 3.58 (s, 2H), 6.76-6.84 (m, 2H), 7.91 (s, breit, 1 H).

### Beispiel 5:

Herstellung von 7-Methoxy-1,3-dihydro-2*H*-indol-2-on 7-Methoxy-3-(methylsulfanyl)-1,3-dihydro-2H-indol-2-on (1.86g) wurde analog zu Beispiel 1 umgesetzt. Man erhielt die Titelverbindung als Feststoff (1.43 g, 95% Ausbeute).
LC-MS: M+H = 164
¹H-NMR (400 MHz, CDCl₃): δ (ppm) = 3.55 (s, 2H), 3.87 (s, 3H), 6.83 (dd, 2H), 6.98 (t, 1 H), 7.83 (s, breit, 1 H).

## Patentansprüche

1. Verfahren zur Herstellung einer Verbindung der Formel (I) 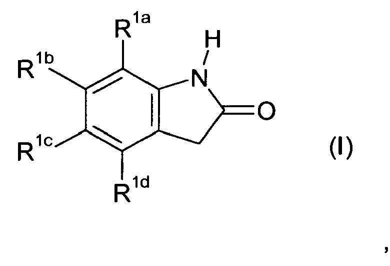 worin
R^{1a} bis R^{1d} unabhängig voneinander ausgewählt sind aus der Gruppe bestehend aus Wasserstofif, Fluor, Chlor, Brom und Trifluormethyl,
(C₁-C₆)-Alkyl, wobei der Alkylrest unsubstituiert ist oder durch einen oder mehrere Substituenten, ausgewählt aus der aus (C₁-C₄)-Alkoxy oder (C₃-C₇)-Cycloalkyl bestehenden Gruppe, substituiert ist,
(C₃-C₇)-Cycloalkyl, wobei der Cycloalkylrest unsubstituiert ist oder durch einen oder mehrere Substituenten, ausgewählt aus der aus (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest verzweigt oder unverzweigt unsubstituiert ist oder durch einen oder mehrere Substituenten, ausgewählt aus der aus (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus (C₁-C₄)-Alkyl oder (C₁-C₄)-Alkoxy bestehenden Gruppe, substituiert ist,
(C₁-C₆)-Alkylthio, wobei der Alkylthiorest verzweigt oder unverzweigt unsubstituiert ist oder durch einen oder mehrere Substituenten, ausgewählt aus der aus (C₁-C₄)-Alkyl oder (C₁-C₄)-Alkoxy bestehenden Gruppe, substituiert ist,
(C₃-C₇)-Cycloalkylthio, wobei der Cycloalkylthiorest unsubstituiert ist oder durch einen oder mehrere Substituenten, ausgewählt aus der aus aus (C₁-C₄)-Alkyl oder (C₁-C₄)-Alkoxy bestehenden Gruppe, substituiert ist, und
Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus (C₁-C₄)-Alkyl, (C₁-C₄)-Alkoxy oder (C₃-C₇)-Cycloalkyl oder (C₁-C₄)-Alkylthio bestehenden Gruppe, substituiert ist,
ausgehend von einer Verbindung der Formel (II) 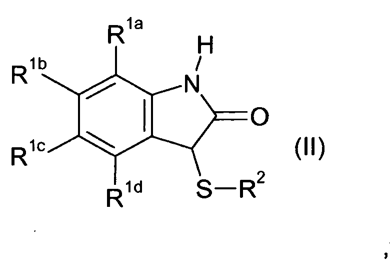 worin
R^{1a} bis R^{1d} wie in Formel (I) definiert ist, und
R² ein unsubstituiertes oder substituiertes (C₁-C₁₄)-Alkyl, (C₃-C₇)-Cycloalkyl, Benzyl oder ein CH₂-C(O)O-(C₁-C₆)-Alkyl ist,
**dadurch gekennzeichnet, dass**
a) eine Verbindung der Formel (II) in einem polaren Lösungsmittel gelöst oder suspendiert wird,
b) der Lösung oder der Suspension ein schwefelhaltiges Salz zugesetzt wird, und
c) das Reaktionsgemisch bei einer Temperatur, die maximal der Siedetemperatur des polaren Lösungsmittels entspricht, unter Rückfluss erwärmt wird.

2. Verfahren zur Herstellung von Verbindungen der Formel (I) nach Anspruch 1, **dadurch gekennzeichnet, dass** R^{1a} bis R^{1d} unabhängig voneinander ausgewählt sind aus der Gruppe bestehend aus Wasserstoff, (C₁-C₆)-Alkyl, (C₁-C₆)-Alkoxy, Fluor, Chlor, Brom und Trifluormethyl.

3. Verfahren zur Herstellung von Verbindungen der Formel (I) nach Anspruch 1 oder 2, **dadurch gekennzeichnet, dass** R^{1a} bis R^{1d} unabhängig voneinander ausgewählt sind aus der Gruppe bestehend aus Wasserstoff, Methoxy, Fluor und Chlor.

4. Verfahren zur Herstellung von Verbindungen der Formel (I) nach einem der Ansprüche 1 bis 3, **dadurch gekennzeichnet, dass** R² ein unsubstituiertes oder substituiertes (C₁-C₆)-Alkyl, (C₃-C₇)-Cycloalkyl, Benzyl oder CH₂-C(O)O-(C₁-C₆)-Alkyl ist, wobei die Substituenten unabhängig voneinander ausgewählt sind aus der Gruppe bestehend aus Wasserstoff, (C₁-C₆)-Alkyl oder (C₃-C₇)-Cycloalkyl.

5. Verfahren zur Herstellung von Verbindungen der Formel (I) nach einem der Ansprüche 1 bis 4, **dadurch gekennzeichnet, dass** das polare Lösungsmittel Wasser, ein (C₁-C₄) Alkohol oder ein Gemisch aus Wasser und einem (C₁-C₄) Alkohol ist.

6. Verfahren zur Herstellung von Verbindungen der Formel (I) nach einem der Ansprüche 1 bis 5, **dadurch gekennzeichnet, dass** das schwefelhaltige Salz ein Alkali- oder Erdalkalisulfit, Alkali- oder Erdalkalibisulfit, Alkali- oder Erdalkalithionit, Alkali- oder Erdalkalidithionit oder Alkali- oder Erdalkalithiosulfat ist.

7. Verfahren zur Herstellung einer Verbindung der Formel (I) nach Anspruch 6, **dadurch gekennzeichnet, dass** das schwefelhaltige Salz ausgewählt ist aus der Gruppe bestehend aus Natriumbisulfit, Natriumsulfit, Natriumthionit, Natriumdithionit und Natriumthiosulfat.

8. Verfahren zur Herstellung einer Verbindung der Formel (I) nach Anspruch 7, **dadurch gekennzeichnet, dass** das schwefelhaltige Salz Natriumsulfit, Natriumbisulfit oder ein Gemisch aus beiden ist.

9. Verfahren zur Herstellung einer Verbindung der Formel (I) nach einem der Ansprüche 6 bis 8, **dadurch gekennzeichnet, dass** dem Reaktionsgemisch 2 bis 3 Äquivalente des schwefelhaltigen Salzes zugesetzt werden.

10. Verfahren zur Herstellung einer Verbindung der Formel (I) nach Anspruch 9, **dadurch gekennzeichnet, dass** dem Reaktionsgemisch 2 bis 2,5 Äquivalente des schwefelhaltigen Salzes zugesetzt werden.

11. Verfahren zur Herstellung einer Verbindung der Formel (I) nach einem der Ansprüche 1 bis 10, **dadurch gekennzeichnet, dass** das Reaktionsgemisch auf eine Temperatur erwärmt wird, die zwischen 40°C und der Rückflusstemperatur des jeweiligen Lösungsmittels liegt.

12. Verfahren zur Herstellung einer Verbindung der Formel (I) nach Anspruch 11, **dadurch gekennzeichnet, dass** die Reaktionstemperatur der Rückflusstemperatur des jeweils eingesetzten Lösungsmittels entspricht.

13. Verfahren zur Herstellung einer Verbindung der Formel (I) nach einem der vorhergehenden Ansprüche, **dadurch gekennzeichnet, dass** die Erwärmung des Reaktionsgemischs unter Rühren für die Zeitdauer von 1 bis 48 Stunden erfolgt.

14. Verfahren zur Herstellung einer Verbindung der Formel (I) nach Anspruch 13, **dadurch gekennzeichnet, dass** die Erwärmung des Reaktionsgemischs unter Rühren für die Zeitdauer von 1 bis 24 Stunden, bevorzugt für die Zeitdauer von 2 bis 12 Stunden erfolgt.

15. Verfahren zur Herstellung einer Verbindung der Formel (I) nach einem der vorhergehenden Ansprüche, **dadurch gekennzeichnet, dass** dem Reaktionsgemisch ein Entschäumer oder eine Mischung verschiedener Entschäumer zugesetzt wird.

16. Verfahren zur Herstellung einer Verbindung der Formel (I) nach einem der vorhergehenden Ansprüche, **dadurch gekennzeichnet, dass** dem Reaktionsgemisch vor dem Isolieren des Reaktionsproduktes gemäß Formel (I) durch Filtration ein Fällungsmittel zugesetzt wird, wobei das Fällungsmittel ausgewählt ist aus der Gruppe der polaren Lösungsmittel bestehend aus Wasser, ein (C₁-C₄) Alkohol oder ein Gemisch aus Wasser und einem (C₁-C₄) Alkohol ist

17. Verfahren zur Herstellung einer Verbindung der Formel (I) nach einem der vorhergehenden Ansprüche, **dadurch gekennzeichnet, dass** das Reaktionsprodukt nach Abschluss der Reaktion durch Filtration isoliert wird.

## Claims

1. Process for preparing a compound of the formula (I) 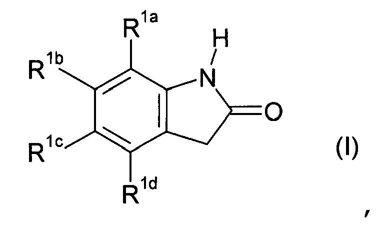 in which
R^{1a} to R^{1d} independently of one another are selected from the group consisting of
hydrogen, fluorine, chlorine, bromine, and trifluoromethyl,
(C₁-C₆)-alkyl, the alkyl radical being unsubstituted or being substituted by one or more substituents selected from the group consisting of (C₁-C₄)-alkoxy or (C₃-C₇)-cycloalkyl,
(C₃-C₇)-cycloalkyl, the cycloalkyl radical being unsubstituted or being substituted by one or more substituents selected from the group consisting of (C₁-C₄)-alkyl or (C₃-C₇)-cycloalkyl or (C₁-C₄)-alkoxy,
(C₁-C₆)-alkoxy, the alkoxy radical being branched or unbranched and being unsubstituted or substituted by one or more substituents selected from the group consisting of (C₁-C₄) -alkoxy or (C₃-C₇)-cycloalkyl,
(C₃-C₇)-cycloalkoxy, the cycloalkoxy radical being unsubstituted or being substituted by one or more substituents selected from the group consisting of (C₁-C₄)-alkyl or (C₁-C₄) -alkoxy,
(C₁-C₆) -alkylthio, the alkylthio radical being branched or unbranched and being unsubstituted or substituted by one or more substituents selected from the group consisting of (C₁-C₄)-alkyl or (C₁-C₄)-alkoxy,
(C₃-C₇)-cycloalkylthio, the cycloalkylthio radical being unsubstituted or being substituted by one or more substituents selected from the group consisting of (C₁-C₄)-alkyl or (C₁-C₄) -alkoxy, and
phenyl or 1-naphthyl or 2-naphthyl or a five- or six-membered heteroaromatic ring having 1 to 2 heteroatoms, the heteroatoms being selected independently of one another from the group consisting of 0 or N, and the aryl or heteroaryl radical being unsubstituted or being substituted by one or more substituents selected from the group consisting of (C₁-C₄)-alkyl, (C₁-C₄)-alkoxy or (C₃-C₇)-cycloalkyl or (C₁-C₄)-alkylthio,
starting from a compound of the formula (II) 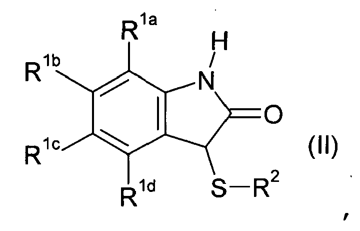 in which
R^{1a} to R^{1d} are defined as in formula (I), and
R² is an unsubstituted or substituted (C₁-C₁₄)-alkyl, (C₃-C₇)-cycloalkyl, benzyl or a CH₂-C(O)O-(C₁-C₆)-alkyl,
**characterized in that**
a) a compound of the formula (II) is dissolved or suspended in a polar solvent,
b) a sulfur-containing salt is added to the solution or suspension,
and
c) the reaction mixture is heated under reflux at a temperature which corresponds at most to the boiling temperature of the polar solvent.

2. Process for preparing compounds of the formula (I) according to Claim 1, **characterized in that** R^{1a} to R^{1d} independently of one another are selected from the group consisting of hydrogen, (C₁-C₆)-alkyl, (C₁-C₆)-alkoxy, fluorine, chlorine, bromine, and trifluoromethyl.

3. Process for preparing compounds of the formula (I) according to Claim 1 or 2, **characterized in that** R^{1a} to R^{1d} independently of one another are selected from the group consisting of hydrogen, methoxy, fluorine, and chlorine.

4. Process for preparing compounds of the formula (I) according to any of Claims 1 to 3, **characterized in that** R² is an unsubstituted or substituted (C₁-C₆)-alkyl, (C₃-C₇)-cycloalkyl, benzyl or CH₂-C(O)O-(C₁-C₆)-alkyl, the substituents being selected independently of one another from the group consisting of hydrogen, (C₁-C₆)-alkyl or (C₃-C₇)-cycloalkyl.

5. Process for preparing compounds of the formula (I) according to any of Claims 1 to 4, **characterized in that** the polar solvent is water, a (C₁-C₄) alcohol or a mixture of water and a (C₁-C₄) alcohol.

6. Process for preparing compounds of the formula (I) according to any of Claims 1 to 5, **characterized in that** the sulfur-containing salt is an alkali metal or alkaline earth metal sulfite, alkali metal or alkaline earth metal bisulfite, alkali metal or alkaline earth metal thionite, alkali metal or alkaline earth metal dithionite or alkali metal or alkaline earth metal thiosulfate.

7. Process for preparing a compound of the formula (I) according to Claim 6, **characterized in that** the sulfur-containing salt is selected from the group consisting of sodium bisulfite, sodium sulfite, sodium thionite, sodium dithionite, and sodium thiosulfate.

8. Process for preparing a compound of the formula (I) according to Claim 7, **characterized in that** the sulfur-containing salt is sodium sulfite, sodium bisulfite or a mixture of both.

9. Process for preparing a compound of the formula (I) according to any of Claims 6 to 8, **characterized in that** 2 to 3 equivalents of the sulfur-containing salt are added to the reaction mixture.

10. Process for preparing a compound of the formula (I) according to Claim 9, **characterized in that** 2 to 2.5 equivalents of the sulfur-containing salt are added to the reaction mixture.

11. Process for preparing a compound of the formula (I) according to any of Claims 1 to 10, **characterized in that** the reaction mixture is heated to a temperature which lies between 40°C and the reflux temperature of the respective solvent.

12. Process for preparing a compound of the formula (I) according to Claim 11, **characterized in that** the reaction temperature corresponds to the reflux temperature of the solvent respectively used.

13. Process for preparing a compound of the formula (I) according to any of the preceding claims, **characterized in that** the heating of the reaction mixture takes place with stirring for a duration of 1 to 48 hours.

14. Process for preparing a compound of the formula (I) according to Claim 13, **characterized in that** the heating of the reaction mixture takes place with stirring for a duration of 1 to 24 hours, preferably for a duration of 2 to 12 hours.

15. Process for preparing a compound of the formula (I) according to any of the preceding claims, **characterized in that** a defoamer or a mixture of different defoamers is added to the reaction mixture.

16. Process for preparing a compound of the formula (I) according to any of the preceding claims, **characterized in that**, prior to the isolation of the reaction product of the formula (I) by filtration, a precipitant is added to the reaction mixture, the precipitant being selected from the group of polar solvents consisting of water, a (C₁-C₄) alcohol or a mixture of water and a (C₁-C₄) alcohol.

17. Process for preparing a compound of the formula (I) according to any of the preceding claims, **characterized in that** the reaction product is isolated by filtration after the reaction is concluded.

## Revendications

1. Procédé pour la préparation d'un composé de formule générale (I) 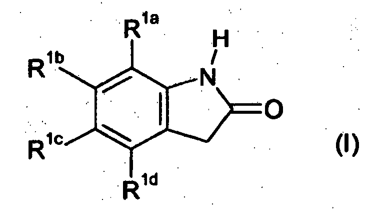 où
R^{1a} à R^{1d} sont choisis, indépendamment les uns des autres, dans le groupe constitué par hydrogène, fluor, chlore, brome et trifluorométhyle,
(C₁-C₆) -alkyle, le radical alkyle étant non substitué ou substitué par un ou plusieurs substituants, choisis dans le groupe constitué par (C₁-C₄)-alcoxy ou (C₃-C₇)-cycloalkyle,
(C₃-C₇)-cycloalkyle, le radical cycloalkyle étant non substitué ou substitué par un ou plusieurs substituants, choisis dans le groupe constitué par (C₁-C₄) -alkyle ou (C₃-C₇)-cycloalkyle ou (C₁-C₄) - alcoxy,
(C₁-C₆)-alcoxy, le radical alcoxy étant ramifié ou non ramifié, non substitué ou substitué par un ou plusieurs substituants choisis dans le groupe constitué par (C₁-C₄) -alcoxy ou (C₃-C₇)-cycloalkyle,
(C₃-C₇)-cycloalcoxy, le radical cycloalcoxy étant non substitué ou substitué par un ou plusieurs substituants, choisis dans le groupe constitué par (C₁-C₄)-alkyle ou (C₁-C₄) -alcoxy,
(C₁-C₆)-alkylthio, le radical alkylthio étant ramifié ou non ramifié, non substitué ou substitué par un ou plusieurs substituants, choisis dans le groupe constitué par (C₁-C₄) -alkyle ou (C₁-C₄) - alcoxy,
(C₃-C₇)-cycloalkylthio, le radical cycloalkylthio étant non substitué ou substitué par un ou plusieurs substituants, choisis dans le groupe constitué par (C₁-C₄) -alkyle ou (C₁-C₄) -alcoxy, et
phényle ou 1-naphtyle ou 2-naphtyle ou un cycle hétéroaromatique de cinq ou six chaînons comprenant 1 à 2 hétéroatomes, les hétéroatomes étant choisis, indépendamment les uns des autres, du groupe constitué par 0 ou N et le radical aryle ou hétéroaryle étant non substitué ou substitué par un ou plusieurs substituants, choisis dans le groupe constitué par (C₁-C₄)-alkyle, (C₁-C₄)-alcoxy ou (C₃-C₇)-cycloalkyle ou (C₁-C₄) -alkylthio,
partant d'un composé de formule (II) 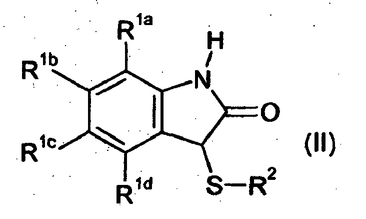 où
R^{1a} à R^{1d} sont tels que définis dans la formule (I) et
R² représente (C₁-C₁₄) -alkyle, (C₃-C₇) -cycloalkyle, benzyle ou CH₂-C(O)O-(C₁-C₆)-alkyle, substitués ou non substitués
**caractérisé en ce que**
a) un composé de formule (II) est dissous ou mis en suspension dans un solvant polaire,
b) la solution ou la suspension est additionnée d'un sel contenant du soufre, et
c) le mélange réactionnel est chauffé à reflux à une température qui correspond au maximum à la température d'ébullition du solvant polaire.

2. Procédé pour la préparation de composés de formule (I) selon la revendication 1, **caractérisé en ce que** R^{1a} à R^{1d} sont choisis, indépendamment les uns des autres, dans le groupe constitué par hydrogène, (C₁-C₆)-alkyle, (C₁-C₆)-alcoxy, fluor, chlore, brome et trifluorométhyle.

3. Procédé pour la préparation de composés de formule (I) selon la revendication 1 ou 2, **caractérisé en ce que** R^{1a} à R^{1d} sont choisis, indépendamment les uns des autres, dans le groupe constitué par hydrogène, méthoxy, fluor et chlore.

4. Procédé pour la préparation de composés de formule (I) selon l'une ou plusieurs des revendications 1 à 3, **caractérisé en ce que** R² représente (C₁-C₆)-alkyle, (C₃-C₇)-cycloalkyle, benzyle ou CH₂-C(O)O-(C₁-C₆)-alkyle, substitués ou non substitués, les substituants étant choisis indépendamment les uns des autres dans le groupe constitué par hydrogène, (C₁-C₆)-alkyle ou (C₃-C₇)-cycloalkyle.

5. Procédé pour la préparation de composés de formule (I) selon l'une quelconque des revendications 1 à 4, **caractérisé en ce que** le solvant polaire est l'eau, un (C₁-C₄)-alcool ou un mélange d'eau et d'un (C₁-C₄) -alcool.

6. Procédé pour la préparation de composés de formule (I) selon l'une quelconque des revendications 1 à 5, **caractérisé en ce que** le sel contenant du soufre est un sulfite de métal alcalin ou alcalino-terreux, un bisulfite de métal alcalin ou alcalino-terreux, un thionite de métal alcalin ou alcalino-terreux, un dithionite de métal alcalin ou alcalino-terreux ou un thiosulfate de métal alcalin ou alcalino-terreux.

7. Procédé pour la préparation d'un composé de formule (I) selon la revendication 6, **caractérisé en ce que** le sel contenant du soufre est choisi dans le groupe constitué par le bisulfite de sodium, le sulfite de sodium, le thionite de sodium, le dithionite de sodium et le thiosulfate de sodium.

8. Procédé pour la préparation d'un composé de formule (I) selon la revendication 7, **caractérisé en ce que** le sel contenant du soufre est le sulfite de sodium, le bisulfite de sodium ou un mélange des deux.

9. Procédé pour la préparation d'un composé de formule (I) selon l'une quelconque des revendications 6 à 8, **caractérisé en ce que** le mélange réactionnel est additionné de 2 à 3 équivalents de sel contenant du soufre.

10. Procédé pour la préparation d'un composé de formule (I) selon la revendication 9, **caractérisé en ce que** le mélange réactionnel est additionné de 2 à 2,5 équivalents de sel contenant du soufre.

11. Procédé pour la préparation d'un composé de formule (I) selon l'une quelconque des revendications 1 à 10, **caractérisé en ce que** le mélange réactionnel est chauffé à une température qui se situe entre 40°C et la température de reflux du solvant en question.

12. Procédé pour la préparation d'un composé de formule (I) selon la revendication 11, **caractérisé en ce que** la température de réaction correspond à la température de reflux du solvant à chaque fois utilisé.

13. Procédé pour la préparation d'un composé de formule (I) selon l'une quelconque des revendications précédentes, **caractérisé en ce que** le chauffage du mélange réactionnel a lieu sous agitation pendant un laps de temps de 1 à 48 heures.

14. Procédé pour la préparation d'un composé de formule (I) selon la revendication 13, **caractérisé en ce que** le chauffage du mélange réactionnel a lieu sous agitation pendant un laps de temps de 1 à 24 heures, de préférence pendant un laps de temps de 2 à 12 heures.

15. Procédé pour la préparation d'un composé de formule (I) selon l'une quelconque des revendications précédentes, **caractérisé en ce que** le mélange réactionnel est additionné d'un antimousse ou d'un mélange de différents antimousses.

16. Procédé pour la préparation d'un composé de formule (I) selon l'une quelconque des revendications précédentes, **caractérisé en ce que** le mélange réactionnel est additionné d'un agent de précipitation avant l'isolement du produit de réaction selon la formule (I) par filtration, l'agent de précipitation étant choisi dans le groupe des solvants polaires constitué par l'eau, un (C₁-C₄)-alcool ou un mélange d'eau et d'un (C₁-C₄)-alcool.

17. Procédé pour la préparation d'un composé de formule (I) selon l'une quelconque des revendications précédentes, **caractérisé en ce que en ce que** le produit de réaction est isolé par filtration après la réaction.
